# EUROPEAN PATENT SPECIFICATION

(11) **EP 2 300 623 B1**
(45) Date of publication and mention of the grant of the patent: **10.04.2013**
(21) Application number: 09754142.9
(22) Date of filing: 27.05.2009
(51) Int. Cl.: C12Q 1/68

(54) **METHOD FOR DETECTING RESPIRATORY VIRAL AGENTS IN A TEST SAMPLE**
VERFAHREN ZUM NACHWEIS VIRALER AGENTIEN DER ATEMWEGE IN EINER TESTPROBE
PROCÉDÉ DESTINÉ À DÉTECTER DES AGENTS VIRAUX DANS UN ÉCHANTILLON DE TEST

(30) Priority: 30.05.2008 GB 0809881
(43) Date of publication of application: 30.03.2011
(73) Proprietor: Genomica S.A.U., 28820 Coslada (ES)
(72) Inventor: VILLAHERMOSA JAEN, Maria L., E-28820 Coslada (ES); MOSCOSO DEL PRADO, Juan, E-28820 Coslada (ES); ALEMAN, Ainel, E-28820 Coslada (ES)
(74) Representative: Fleck, Barbara
(86) International application number: PCT/GB2009/050574
(87) International publication number: WO 2009/144497

(56) References cited:
- WO-A-2004/057021
- DATABASE EMBL [Online] 29 June 2004 (2004-06-29), "Human MPV strain fusion NH456-02 glycoprotein gene, partial c" XP002543735 retrieved from NCBI Database accession no. GenBank AY622280
- DATABASE EMBL [Online] 29 June 2004 (2004-06-29), "Human MPV strain fusion NH708-02 glycoprotein gene, partial cds" XP002543736 retrieved from NCBI Database accession no. GenBank AY622306
- DATABASE EMBL [Online] 8 October 2003 (2003-10-08), "hMTV isolate HK387 RNA polymerase (L) dene, partial cds" XP002543737 retrieved from NCBI Database accession no. GenBank AY294866
- DATABASE EMBL [Online] 9 April 2004 (2004-04-09), "hMPV isolate TN/85-3-1 RNA polymerase (L) gene, partial cds" XP002543738 retrieved from NCBI Database accession no. GenBank AY216971
- MAERTZDORF JEROEN ET AL: "Real-time reverse transcriptase PCR assay for detection of human metapneumoviruses from all known genetic lineages." JOURNAL OF CLINICAL MICROBIOLOGY MAR 2004, vol. 42, no. 3, March 2004 (2004-03), pages 981-986, XP002543733 ISSN: 0095-1137
- MACKAY IAN M ET AL: "Use of the P gene to genotype human Metapneumovirus identifies 4 viral subtypes" JOURNAL OF INFECTIOUS DISEASES, vol. 190, no. 11, 1 December 2004 (2004-12-01), pages 1913-1918, XP002543734 ISSN: 0022-1899
- VAN DEN HOOGEN B G ET AL: "Analysis of the Genomic Sequence of a Human Metapneumovirus" VIROLOGY, ACADEMIC PRESS,ORLANDO, US, vol. 295, no. 1, 30 March 2002 (2002-03-30), pages 119-132, XP004467149 ISSN: 0042-6822

## Description

### FIELD OF THE INVENTION

The present invention relates to claim 1 and claim 8. Also described are the detection of respiratory viruses Influenza A, Influenza B, Influenza C, PIV 1, PIV 2, PIV 3, PIV 4A, 4B, Adenovirus, hRSV A, hRSV B, Coronavirus229, Echovirus 30, Rhinovirus and hBoV. The invention also relates to claim 12 and claim 13.

We also describe a set of nucleic acid sequences, as well as to their use as amplification primers for hMPV present in a test sample. Amplification of hMPV with the nucleic acid sequences according to the present invention is compatible with determining the hMPV genotype present in the test sample.

### BACKGROUND OF THE INVENTION

Respiratory tract infections are a significant cause of morbidity and mortality in all age groups but especially in young children, elderly subjects, and immunocompromised patients. Most of these infections are caused by viruses.

Further to the most common viruses that cause respiratory infections, recent studies suggest that human metapneumovirus (hMPV) should be added to the list of human respiratory viral pathogens in all age groups (van den Hoogen et al., 2001, Nat. Med. 7:719-724; Peret et al., 2002, J. Infect. Dis. 185:1660-1663; Boivin et al., 2002, J. Infect. Dis.186:1330-1334).

As an example of a prospective study, in an analysis of 1,322 hospitalized infants <2 years of age in Spain through 5 years, hMPV was found to be the most common virus after hRSV and rhinovirus (Garcia-Garcia et al., 2006, Pediatr. Pulmonol. 41 (9):863-71). Co-infections were frequent and clinically similar to single infections and hRSV infections. Further data can be found at Garcia-Garcia et al., 2006, Arch Dis Child. 91 (4):290-5.

Recently, a higher virulence of hMPV genotype A with respect to B has been suggested (Vicente et al., 2006, Clin Infect Dis. 42(12):e111-3). The clinical spectrum of 69 episodes of metapneumovirus pediatric infection (55 episodes caused by genotype A and 14 episodes caused by genotype B) was analysed, and it was found that diagnosis of pneumonia was more common and the illness severity index (determined on the basis of need for hospitalization, oxygen saturation <90%, and intensive care unit stay) was higher for patients with hMPV genotype A infection. The different severity observed could be attributable to a higher virulence of hMPV genotype A (hMPV-A) with respect to B (hMPV-B).

Attempts for the simultaneous detection of hMPV and other viral agents affecting respiratory tract have already been described (WO2005/038427 and WO2006/070034 constitute representative examples). A comprehensive list of respiratory viruses includes: Influenza A and B, human respiratory syncytial virus (hRSV) A and B, the four serotypes of human parainfluenza viruses (PIV), and adenovirus, rhinoviruses, coronaviruses, Influenza C, as well as enteroviruses. Further, the knowledge of viruses that cause lower respiratory tract infections has recently been enriched with the characterization of a new member, Human Bocavirus (HBoV) (Allander et al., 2005, PNAS 102: 12891-12896).

Classic amplification primers for hMPV are L6 and L7, first described in Van den Hoogen et al., 2003, J. Infect. Dis. 188:1571-1577. Some other state of the art examples where L6 and L7 have been used for hMPV amplification are WO2005/038427 and W02006/070034.

In particular, WO2005/038427 provides a method for amplification of target sequences of viruses of the upper respiratory tract, the method including PCR amplification using multiple pairs of PCR primers. As amplification primers for hMPV, primers L6 and L7 are used.

Further, in WO2006/070034, amplification of respiratory viruses present in a sample may be carried out prior to hybridization of the amplification products with virus-specific probes. The primers used for amplification of hMPV are again L6 and L7.

Further patent applications of the state of the art directed to the simultaneous detection of respiratory viral pathogens are:

US2007/092871, which describes microarrays having a plurality of oligonucleotide probe sequences for the genetic identification of upper respiratory pathogens. As regards primers disclosed in the application, only sequences corresponding to a pool of PCR primers for amplifying influenza A samples are provided.

CA2418004, which describes an assay for detection in a test sample nucleic acids from clinically important respiratory viral pathogens in a multiplex format. Most of the amplification primer sequences displayed in the patent application had been previously described by others, with the exception of those of hMPV, which were first described in the patent application. However, these sequences corresponding to the hMPV amplification primers differ from those described herein.

WO2005/005658, which discloses a detection method for the coronavirus causing the severe acute respiratory syndrome (SARS-CoV), the method based on a chip which comprises a support wherein oligonucleotide probes are immobilized. Further to the chip, the patent application further discloses some oligonucleotide primers for amplifying other respiratory viruses, including some for amplification of human metapneumovirus. However, none of the amplification primers of patent application WO2005/005658 correspond to those described herein.

WO2004/057021, which discloses compositions and methods for the detection of respiratory viruses by means of the use of specific nucleic acid sequences.

None of the hMPV amplification primers disclosed in WO2004/057021 correspond to those disclosed within the invention described herein.

WO2006/102695, which discloses the use of real time polymerase chain reaction (PCR) for the detection of respiratory infectious organisms. Some sequences corresponding to primers as well as to anchor and sensor probes are provided therein. As regards the amplification primers corresponding to HMPV, the sequences provided differ from those of the present invention.

WO2004/096993, which provides methods for detecting a mammalian metapneumovirus in a sample, wherein the method comprises either contacting the sample with a nucleic acid probe, or else contacting the sample with an antibody, or alternatively, amplification of the MPV nucleic acid of the sample. As regards this latter detection method, it is generally stated that amplification primers may specifically hybridize to the nucleic acid sequence of the human metapneumovirus DNA, without provision of specific primer sequences.

### SUMMARY OF THE INVENTION

The problem to be solved by the present invention is the provision of a method which allows an alternative or even more efficient detection of hMPV in a test sample than methods of the state of the art. Preferably, the method should be compatible with the subsequent typing of the hMPV present, and with detection of other respiratory viruses present in the sample.

The solution, which corresponds to a first aspect of the present invention, is based on one or more nucleic acid sequences comprising a sequence selected from the group comprising SEQ ID Nº 1-6, their complements and equivalents thereof, and to their use as amplification primers in hMPV amplification. By equivalents is meant sequences that may differ from the given sequence, for example by at least one, preferably one, two, three, four mutations including alterations, deletions and insertions. Other equivalents may be primers incorporating alternative or modified nucleotides in place of canonical nucleotides; provided that the equivalent sequence can act as amplification primers in hMPV amplification. Nucleic acid includes, for example, DNA, RNA and PNA and any other form or modification. Thus, the invention relates to claim 1.

Nucleic acid sequences comprising sequences SEQ ID Nº 1 and 2, and sequences SEQ ID Nº 3 and 4, and SEQ ID Nº 5 and 6, or their complements and equivalents thereof, constitute pairs of amplification primers for hMPV.

Amplification of test samples with primers according to the present invention results in a more sensitive detection of hMPV than that of the state of the art, both in agarose gel electrophoresis, as well as in detection systems based on hybridization with target-specific probes.

The state of the art shows a widespread interest in detection methods of respiratory viruses by means of nucleic acid amplification. A skilled person using methods known in the art to generate amplification primers for hMPV would produce many sequences, the effectiveness of which would be unknown and impossible to test within a reasonable time frame and with reasonable resources.

The method of the present invention allows a more efficient detection than methods of the state of the art of hMPV present in a test sample. Thus, amplification with primers L6.2 and L7.2 (SEQ ID Nº 1 and 2, respectively) allows detection of hMPV in samples where amplification with L6 and L7 of the state of the art (SEQ ID Nº 7 and 8, respectively) provided a negative result.

Further, amplification with primers SA, AA, SB and AB (SEQ ID Nº 3 to 6, respectively), followed by hybridization with a sequence-specific probe, such as probes F249B, F256B or F256A (SEQ ID Nº 11, 12 and 13, respectively), complemented the detection ability of a detection method comprising amplification with primers L6 and L7 and L6.2 and L7.2, followed by hybridization with probes HMNVA-S37 and HMNVB-S38 (SEQ ID Nº 9 and 10, respectively) up to detection of almost 100% hMPV positive samples.

Further, the method of amplification with primers L6.2 and L7.2 either in the presence or absence of primers L6 and L7 is compatible with amplification in the presence of pairs of amplification primers for Influenza A, Influenza B, Influenza C, PIV 1, PIV 2, PIV 3, PIV 4A, 4B, Adenovirus, hRSV A, hRSV B, Coronavirus229, Echovirus 30, Rhinovirus and hBoV.

An advantage of the present invention is that hMPV amplification with the pairs of primers of the invention, is compatible with genotyping of hMPV by way of hybridization of the amplification products, previously transformed into single-stranded oligo- or polynucleotides, with the probes of the present invention. Thereby, no nested amplification is required for genotyping.

Another aspect of the present invention corresponds to claim 6.

The first mixture of amplification primers may additionally comprise primers L6 (SEQ ID Nº 7) and L7 (SEQ ID Nº 8).

Additionally, the kit of the present invention may further allow detection of Influenza A, Influenza B, Influenza C, PIV 1, PIV 2, PIV 3, PIV 4A, 4B, Adenovirus, hRSV A, hRSV B, Coronavirus229, Echovirus 30, Rhinovirus and hBoV, in a test sample, the kit preferably comprising:
- Pairs of primers of SEQ ID Nº 14-24 for PIV 1, PIV 2, PIV 3, PIV 4A, 4B, hRSV A and Coronavirus type 229, within the first amplification mixture;
- and pairs of primers of SEQ ID Nº 25-38 for Influenza A, Influenza B, Influenza C, hRSV B, Adenovirus, Echovirus 30, Rhinovirus and hBoV, within the second amplification mixture.

Further to the amplification mixtures, the kit may further comprise:
i) an array vessel or set of array vessels, each comprising a microarray wherein target-specific probes are provided; and
ii) reagents for use in visualising hybridisation of nucleic acids to the probes of the microarray.

We also described the use of the above-mentioned method and kit, for the detection and identification, if present in a test sample, of one or more viral agents selected from the group comprising hMPV, Influenza A, Influenza B, Influenza C, PIV 1, PIV 2, PIV 3, PIV 4A, 4B, Adenovirus, hRSV A, hRSV B, Coronavirus229, Echovirus 30, Rhinovirus and hBoV.

Further aspects of the present invention correspond to claim 12 and claim 13. We also described (i) a nucleic acid amplification mixture comprising, as amplification primers, nucleic acids comprising SEQ ID Nº 1 and 2, and/or nucleic acids comprising SEQ ID Nº 3 and 4, and/or nucleic acids comprising SEQ ID Nº 5 and 6, or their complements and equivalents thereof. Preferably the mixture comprises as amplification primers first to fourth nucleic acids comprising SEQ ID NO 3-6 respectively. The mixture may also comprise fifth and sixth nucleic acids comprising SEQ ID NO 1 and 2 respectively, but in preferred embodiments, the fifth and sixth nucleic acids are not included in the same mixture as the first to fourth. (ii) Amplification fragments obtainable with the pairs of primers of SEQ ID Nº 1 and 2; 3 and 4; and 5 and 6; and (iii) A method for producing nucleic acid sequences comprising SEQ ID Nº 1-6, their complements and equivalents thereof, wherein the nucleic acid sequences may be labelled. Preferably this label is biotin.

### BRIEF DESCRIPTION OF THE DRAWINGS

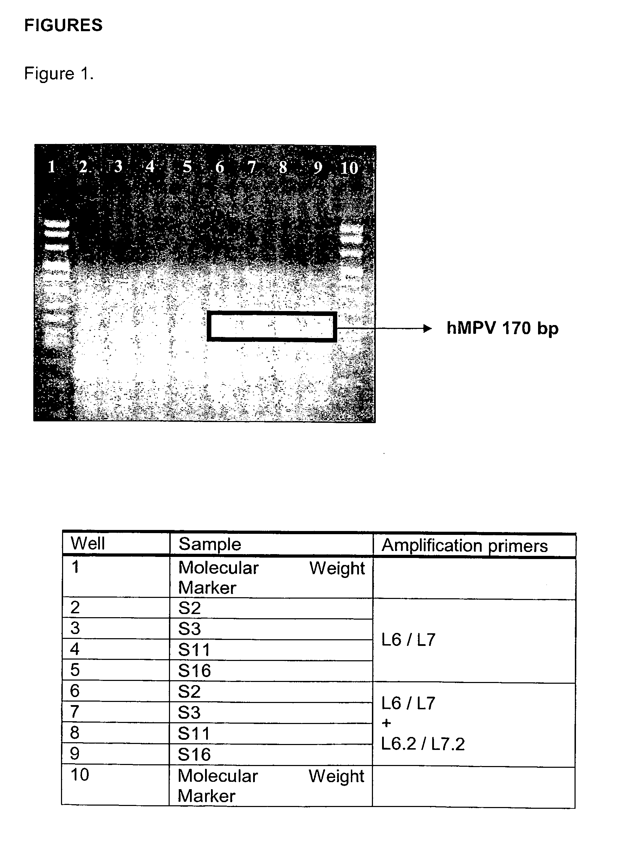

Figure 1. Agarose gel visualization of amplification products obtained by RT-PCR amplification reaction with the pair of amplification primers L6 (SEQ ID Nº 7) and L7 (SEQ ID Nº 8), in the absence (Lanes 2-5) or presence (Lanes 6-9) of the pair of amplification primers L6.2 (SEQ ID Nº 1) and L7.2 (SEQ ID Nº 2).

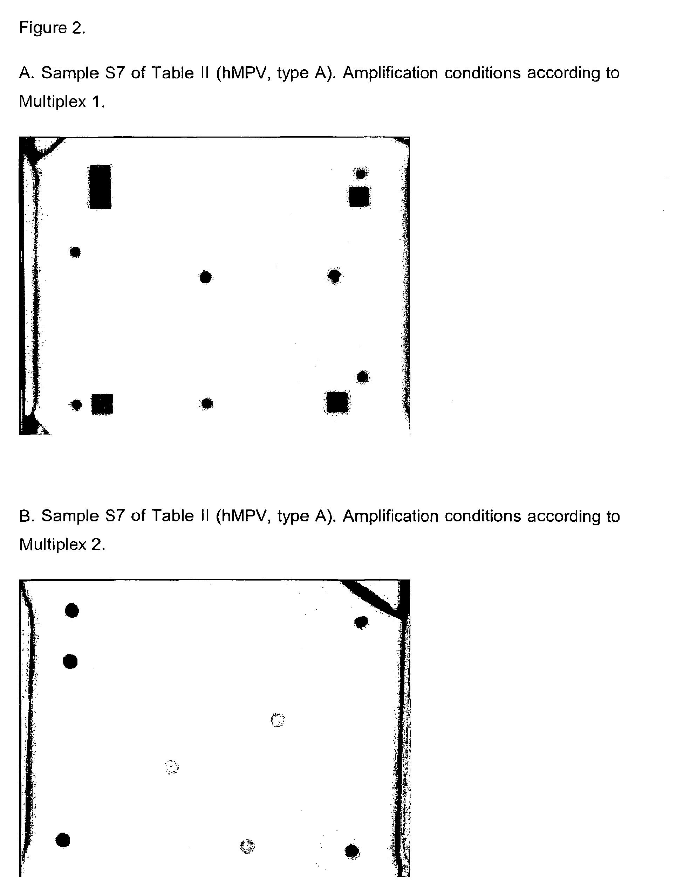

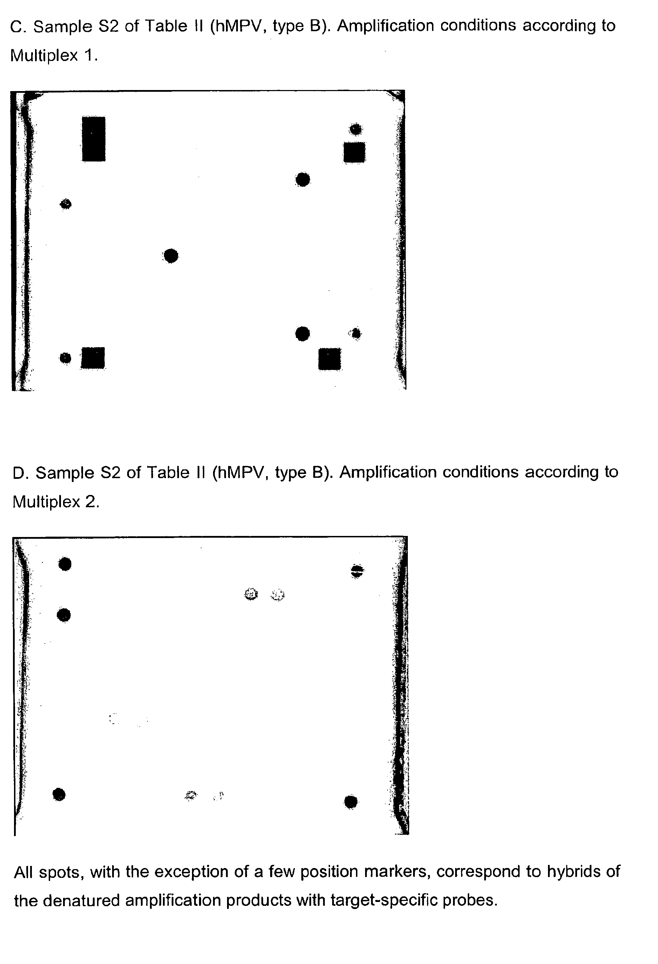

Figure 2. Results of Array Tubes corresponding to sample S7 (hMPV-A positive), subjected to amplification conditions according to Multiplex 1 (Panel A) and Multiplex 2 (Panel B), and to sample S2 (hMPV-B positive), subjected to amplification conditions according to Multiplex 1 (Panel C) and Multiplex 2 (Panel D).

Definitions. The following terms have the indicated meanings in the specification unless expressly indicated to have a different meaning:
- Amplification Primers: Nucleic acids that bind to and allow amplification of one or more target sequences.
- Array tube: An individual array vessel which has a shape and size typical of a laboratory reaction vessel (for example, a 1.5 ml Eppendorf tube) with a microarray arranged therein in which microarray based tests can be carried out.
- Array vessel: A reaction vessel with flat bottom comprising a microarray. The probe molecules of the microarray may be printed on a solid support wherein this solid support can be the bottom of an array vessel, or a different solid support attached to the bottom of an array vessel.
- Equivalent: Sequence of nucleotides correspondent to that of a given sequence of DNA, wherein one or more of the nucleotides are ribonucleotides, or modified nucleotides such as inosine, which do not essentially alter the hybridization characteristics.
- Labelling: Introduction of a modification group within a nucleic acid sequence.
- Microarray: Arrangement of molecular probes on a surface, wherein the position of each probe is separately determined.
- Multiplex PCR and RT-PCR reactions: PCR and RT-PCR reactions that allow amplification of two or more nucleic acid sequences if present.
- Probes: Nucleic acid with ability to specifically bind to a target nucleic acid sequence. This includes DNA, RNA, PNA, and any other form or modification.
- Sensitivity of detection: Minimum number of copies detected. "Higher or Improved sensitivity" here means that there is a lower minimum detectable copy number.
- Strip of vessels: A set of array vessels, usually 8, each with a microarray arranged therein, in which microarray based tests can be carried out.
- Target sequences: Sequences to be detected.
- Target-specific probes: Probes that hybridize specifically with the target sequences amplifiable in the amplification reactions.

### DETAILED DESCRIPTION OF THE INVENTION

The present invention relates to a set of nucleic acid primes comprising sequences SEQ ID Nº 1-6 (Table 1). We also described, their complements and equivalents thereof.

**Table I**

| SEQ ID Nº | Primer Name | Sequence (5'-3') |
|---|---|---|
| 1 | L6.2 | TATGCCTACTATAAAAGGTCA |
| 2 | L7.2 | CACCCCAGTCTTTCCTAAAG |
| 3 | SA | GAGAATCCCAGACAATCTAG |
| 4 | AA | ACTYTCAAGCCGGATGGTT |
| 5 | SB | GAAAATCCCAGACAATCAAG |
| 6 | AB | ACTCTCAAGCCTTATRGTT |
| 7 | L6 | CATGCCCACTATAAAAGGTCAG |
| 8 | L7 | CACCCCAGTCTTTCTTGAAA |

Nucleotides of the sequences are designated as follows: G for Guanine, A for Adenine, T for Thymine, C for Cytosine, R for G or A, Y for T or C, M for A or C, K for G or T, S for G or C, W for A or T, H for A or C or T, B for G or T or C, V for G or C or A, D for G or A or T, and finally, N for G or A or T or C.

We also described nucleic acids that comprise sequences selected from the group comprising SEQ ID Nº 1-6 of Table I, their complements and equivalents thereof, further comprising 1, 2, 3, 4, 5, 6, 7, 8, 9 or 10 extra nucleotides, either at the 5', 3' or both 5' and 3' ends. In another preferred embodiment, they consist of the sequences SEQ ID Nº 1-6 of Table I.

The nucleotides as used in the present invention may be deoxyribonucleotides, but also ribonucleotides, modified nucleotides such as inosine, and nucleotides containing modification groups, as long as the hybridization characteristics are not essentially altered. These variations are all included in the term "equivalent". Preferably an equivalent includes one or more modified nucleotides. Alternatively, or in addition, an equivalent includes one or more non-naturally occurring nucleotide. In other embodiments an equivalent is RNA.

In a preferred embodiment the nucleic acid sequences of the present invention are labelled. In a more preferred embodiment, they are labelled with biotin.

We also describe the use of one or more nucleic acid sequences as previously described, as amplification primers in hMPV amplification. In a preferred embodiment of the present invention, nucleic acid sequences comprising sequences SEQ ID Nº 1 and 2, and/or sequences SEQ ID Nº 3 and 4, and/ or SEQ ID Nº 5 and 6, or their complements and equivalents thereof, constitute pairs of amplification primers for hMPV. one or both primers of each pair of amplification primers may be labelled.

Amplification products from different test samples, obtained with the pair of amplification primers of the state of the art L6 and L7 (SEQ ID Nº 7 and 8, respectively, of Table I), in the absence or presence of the pair of amplification primers L6.2 and L7.2 (SEQ ID Nº 1 and 2, respectively, of Table I), were compared as in Example 1 below. Visualization of amplification products was determined by agarose gel electrophoresis. As can be observed in Figure 1, amplification with primers L6.2 and L7.2 resulted in detection of hMPV present in the sample, while in their absence, the presence of hMPV was not detected.

One aspect of the present invention relates to claim 1

Nucleic acid sequences comprising sequences SEQ ID Nº 1 and 2, and sequences SEQ ID Nº 3 and 4, and SEQ ID Nº 5 and 6, or their complements and equivalents thereof, constitute pairs of amplification primers for hMPV.

Amplification of a number of test samples was carried out with the sets of amplification primers:
- L6 and L7, in the absence of L6.2 and L7.2;
- L6 and L7, in the presence of L6.2 and L7.2; and
- SA, AA, SB and AB (of respective SEQ ID Nº 3, 4, 5, and 6).

A representative example of adequate amplification conditions corresponding to this comparative study is to be found in Example 2.

The amplification products thus obtained, were transformed into single-stranded oligo- or polynucleotides, followed by hybridization with target-specific probes corresponding to the amplification fragments, and visualization of the results. Some illustrative images are displayed in Figure 2. Sensitivity of detection values of this approach are usually an order of magnitude higher than those of agarose gel electrophoresis.

Table II displays results corresponding to a number of samples tested according to the experimental condictions described under Example 2.

**Table II**

| | Pairs of amplification primers. | | |
|---|---|---|---|
| Sample | L6/L7 | L6/L7 + L6.2/L7.2 | SA/AA + SB/AB |
| S1 | - | hMPV-B | hMPV-B |
| S2 | - | hMPV-B | hMPV-B |
| S3 | - | hMPV-B | hMPV-B |
| S4 | - | - | - |
| S5 | - | hMPV-B | - |
| S5 | - | hMPV-A | hMPV-A |
| S7 | hMPV-A | hMPV-A | hMPV-A |
| S8 | - | hMPV-A | - |
| S9 | hMPV-A | hMPV-A | - |
| S10 | hMPV-A | hMPV-A | - |
| S11 | hMPV-B | hMPV-B | hMPV-B |
| S12 | - | hMPV-A | - |
| S13 | hMPV-A | hMPV-A | - |
| S14 | - | - | hMPV-B |
| S15 | hMPV-A | hMPV-A | - |
| S16 | hMPV-B | hMPV-B | hMPV-B |
| S17 | hMPV-A | hMPV-A | - |
| S18 | - | hMPV-A | hMPV-A |
| S19 | - | hMPV-B | hMPV-B |
| S20 | hMPV-A | hMPV-A | hMPV-A |
| S21 | - | hMPV-B | hMPV-B |
| S22 | hMPV-A | - | hMPV-A |

Amplification products obtained with the pair of amplification primers L6 and L7, both in the absence and in the presence of L6.2 and L7.2, were denatured and hybridized with newly designed probes HMNVA-S37 and HMNVB-S38, which respectively detect genotypes A and B of hMPV.

| SEQ ID Nº | Probe Name | Sequence (5'-3') |
|---|---|---|
| 9 | HMNVA-S37 | GAGTCTTTRTCAGCAGCRTTAGC |
| 10 | HMNVB-S38 | GAATCTTTATCTGCAGCACTTGC |

Nucleotides of the sequences are designated as follows: G for Guanine, A for Adenine, T for Thymine, C for Cytosine, R for G or A, Y for T or C, M for A or C, K for G or T, S for G or C, W for A or T, H for A or C or T, B for G or T or C, V for G or C or A, D for G or A or T, and finally, N for G or A or T or C.

Hybridization of the amplification products with a generic probe of the state of the art for hMPV, such as (TGGTGTGGGATATTAACAG), did not provide a different result.

Amplification products obtained with the pairs of amplification primers SA/AA and SB/AB, were denatured and hybridized with newly designed probes:

| SEQ ID Nº | Probe Name | Sequence (5'-3') | Genotype |
|---|---|---|---|
| 11 | F249B | CAG CAG CAG TCA CAG CAG GCA TT | hMPV-B |
| 12 | F256B | GTC ACA GCA GGC ATT GCR ATA GC | hMPV-B |
| 13 | F256A | GTT ACA GCA GGT GTT GCA ATT GC | hMPV-A |

Nucleotides of the sequences are designated as follows: G for Guanine, A for Adenine, T for Thymine, C for Cytosine, R for G or A, Y for T or C, M for A or C, K for G or T, S for G or C, W for A or T, H for A or C or T, B for G or T or C, V for G or C or A, D for G or A or T, and finally, N for G or A or T or C.

As can be inferred from Table II, the method of the present invention allows a more efficient detection of hMPV present in a test sample than methods of the state of the art. Thus, amplification with primers L6.2 and L7.2 allows detection of hMPV in samples where amplification with L6 and L7 provided a negative result.

Further, amplification with primers SA, AA, SB and AB, and hybridization with probes F249B, F256B and F256A, complemented the detection ability of primers L6.2 and L7.2, up to detection of almost 100% hMPV positive samples.

Another advantage of the method of the present invention over existing methods of the state of the art, is that it can distinguish between types A and B of hMPV without sequencing and/or analysis with restriction enzymes. Further, amplification of hMPV is compatible with the presence of amplification primers for the additional respiratory viruses Influenza A, Influenza B, Influenza C, PIV 1, PIV 2, PIV 3, PIV 4A, 4B, Adenovirus, hRSV A, hRSV B, Coronavirus229, Echovirus 30, Rhinovirus and hBoV (see Example 2 below as a representative example).

In a preferred embodiment of the present invention, the first mixture of amplification primers further comprises primers of SEQ ID Nº 7 and 8.

We also describe a method that further comprises contacting the sample with one or more amplification primers for one or more viral agents selected from the group comprising Influenza A, Influenza B, Influenza C, PIV 1, PIV 2, PIV 3, PIV 4A, 4B, Adenovirus, hRSV A, hRSV B, Coronavirus229, Echovirus 30, Rhinovirus and hBoV.

For example the amplification primers corresponding to Influenza A, Influenza B, Influenza C, PIV 1, PIV 2, PIV 3, PIV 4A, 4B, Adenovirus, hRSV A, hRSV B, Coronavirus229, Echovirus 30, Rhinovirus and hBoV are as follows:

| SEQ ID Nº | Primer Name | Sequence (5'-3') |
|---|---|---|
| 14 | 1PIV13 | AGG WTG YSM RGA TAT AGG RAA RTC ATA |
| 15 | Piv1-A-218 | CAC AGT GGG CAR GGA GCA TAA |
| 16 | 3PIV24 | CYM AYG GRT GYA YTM GAA TWC CAT CAT T |
| 17 | 4PIV4 | TGA CTA TRC TCG ACY TTR AAA TAA GG |
| 18 | 4PIV2 | GCT AGA TCA GTT GTG GCA TAA TCT |
| 19 | Piv3-S-118 | CAA TAG RAA GTC ATG TTC TCT |
| 20 | Piv-3-A-313 | TGG TCC AAC AGA TGG GTA TAA TGC C |
| 21 | RSVA-S-15 | CCT CAA ARC AAA TGC AAT TAC CG |
| 22 | RSVA (2) 3? | TAT ACC AAC CAG TTC TTA GAG C |
| 23 | 1HCoV | TGT GCC ATA GAR GAY WTA CTT TTT |
| 24 | Cor229-A-214 | GTA TTG AAR CGG CTG ATG TTA G |
| 25 | NPAC11 | GAA CTC RTC CYW WAT SWC AAW GRR GAA AT |
| 26 | FluA-A-241 | AAC TCC AWY ACC ATT GTC CC |
| 27 | NPB1 | ACA GAG ATA AAG AAG AGC GTC TAC AA |
| 28 | FluB-A-136 | TGT CAT CAG TGG CAG CCA ATA G |
| 29 | RSVB-541 | TCC AYA GRT CAA GTG CAA TCT TCC T |
| 30 | RSVB 3? | AAA GCA CTA AAA TAA CCT CTG C |
| 31 | ADV1 F+ | CAA CAC CTA YGA STA CAT GAA |
| 32 | ADV2R- | ACA TCC TTB CKG AAG TTC CA |
| 33 | HER1 | CTC CGG CCC CTG AAT RYG GCT AA |
| 34 | HER4 | CTG TGT TGA WAC YTG AGC ICC CA |
| 35 | FIuC-S-122 | CGC AGC AAG AAG AAA CGG TT |
| 36 | FluC-A-217 | GCG TCA GCT ATA AGA ACY CCA ATT C |
| 37 | NS1-1545S | TAT GGC CAA GGC AAT CGT CCA AG |
| 38 | NS1-A1835 | GCC GCG TGA ACA TGA GAA ACA GA |

Nucleotides of the sequences are designated as follows: G for Guanine, A for Adenine, T for Thymine, C for Cytosine, R for G or A, Y for T or C, M for A or C, K1 for G or T, S for G or C, W for A or T, H for A or C or T, B for G or T or C, V for G or C or A, D for G or A or T, and finally, N for G or A or Tor C.

In a preferred embodiment the method comprises:
a) dividing the test sample into two or more samples;
b) contacting one sample with a first mixture of amplification primers, said mixture comprising both nucleic acid sequences comprising sequences SEQ ID Nº 1 and 2, or their complements or equivalents thereof, and primers of SEQ ID Nº 7 and 8, as amplification primers for hMPV, and pairs of amplification primers of SEQ ID Nº 14-24 as amplification primers for PIV 1, PIV 2, PIV 3, PIV 4A, 4B, hRSV A and Coronavirus type 229;
c) contacting another sample with a second mixture of amplification primers comprising nucleic acid sequences comprising sequences SEQ ID Nº 3 and 4, and SEQ ID Nº 5 and 6, or their complements and equivalents thereof, as amplification primers for hMPV, and pairs of amplification primers of SEQ ID Nº 25-38 as amplification primers for Influenza A, Influenza B, Influenza C, hRSV B, Adenovirus, Echovirus 30, Rhinovirus and hBoV; and
d) subjecting the different sets of test samples mixed with amplification primers to amplification reactions being intended to amplify target sequences present in the sample.

In a preferred embodiment, the test sample consists of genetic material extracted from the original sample. Extraction of the genetic material can be carried out both by automatic as well as by manual extraction techniques of the state of the art.

In a most preferred embodiment, the automatic extraction system is the NucliSENS easyMAG of BioMerieux (EP1694813), which uses magnetic particles in combination with BioMerieux's BOOM® technology for universal isolation of total nucleic acid from a wide range of sample volumes and types.

The skilled person will be aware of techniques and methods for manual processing of samples to extract nucleic acids from a sample; and any such suitable method may be used.

In a particular example, nucleic acids are extracted from 50 µl of test sample. After the extraction step, a precipitate is resuspended in 20-25 µl RNase-free water.

In a preferred/ particular embodiment, 5 µl of the 20-25 µl comprising the genetic material extracted from the test sample, are added to the vessel containing the amplification reactants, in a final volume of 50 µl.

An inherent problem of multiplex PCRs is that the presence of several primers in the same amplification reaction could lead to an interaction among the primers present, that might preclude their hybridisation with their target sequences and corresponding amplification. This technical hurdle was overcome by the specific combination of amplification primers of the Multiplexes 1 and 2.

In a preferred embodiment, the amplification reaction is RT-PCR.

In another preferred embodiment, one or more amplification primers of each pair of amplification primers are labelled. In particular, with biotin.

Alternatively, a label is introduced in the amplification product during amplification.

In a preferred embodiment, the method described herein further comprises obtaining single-stranded oligo- or polynucleotides from any amplification products, allowing such single-stranded oligo- or polynucleotides to hybridise with a plurality of target-specific probes, and detecting hybridised oligo- or polynucleotides. More preferably, single-stranded oligo- or polynucleotides are obtained by denaturing any double-stranded oligo- or polynucleotides present.

In a preferred embodiment the RT-PCR products obtained with the amplification primers according described herein, can be characterized by means of microarray technology. The microarray technology provides the simultaneous detection of multiple molecular markers for diagnostic use, while providing the controls needed to ensure the reliability of the results.

In a preferred embodiment, a label may be introduced in the amplified product during its amplification to allow further detection, preferably a label that provides a signal that may be detected by colorimetric methods. In the most preferred embodiment, the label is biotin. However, any other kind of label known in the art may be used (e. g. digoxigenin). Radioactive labels may be used, or fluorophores, in certain embodiments. In a preferred embodiment, labelling of amplified product may be achieved by adding modified nucleotides bearing a label (e. g. biotinylated or digoxigenin dUTP derivatives) in the amplification mixture. In another even more preferred embodiment, the label is contained in the amplification primers.

In a preferred embodiment, amplified product previously denatured is hybridized with target-specific probes to minimise the problem of multiplex PCR, which often results in the production of aspecific amplification fragments, due to the presence of several primers.

In a preferred embodiment, denaturing of amplified DNA can be performed by heating. Other ways to prepare single stranded DNA after amplification may be used instead or as well; for example, chemical means.

In a preferred embodiment, the single stranded DNA is incubated with a plurality of target-specific probes provided on a microarray. At least one, but preferably more than one probe with ability to hybridise with each target sequence, are provided on the microarray. In certain embodiments the single stranded DNA may be incubated with target-specific probes provided in solution; However, it is preferred that the probes are provided on a solid support.

In a preferred embodiment, the probes are contained in a microarray, which may be placed on a slide or contained in a reaction vessel, which is then called an array vessel. Array vessels may have different formats of presentation, including individual array vessels, in particular, array tubes, or sets of array vessels arranged in strips or flat plates. Usually, plates consist of sets of strips of array vessels. Thus, a microarray described herein may be contained in an individual array vessel. Alternatively, two or more microarrays may be contained in a strip of vessels. In a preferred embodiment, the strip of vessels is constituted by 8 vessels. Further, three or more array vessels may be arranged in a set of strip of vessels. In another preferred embodiment, the set of strip of vessels is a microtiter plate.

In preferred embodiments, the probe molecules of the microarray may be printed on a solid support wherein this solid support can be the bottom of an array vessel or a different solid support attached to the bottom of an array vessel. This means that the surface of the microarray may be the flat bottom of the array vessel. Alternatively, the surface of the microarray may be a solid support attached to the bottom of the array vessel.

In an embodiment, the reaction vessel has a typical size for a laboratory reaction vessel. Typical filling volumes lie in the range of 100 µl to 2.5 ml, but can also be higher or lower in special embodiments. Especially preferably the reaction vessel is an array tube. i.e. an array vessel with a normal filling volume for a standard Eppendorf tube of up to 1.5 ml. Further preferred filling volumes are up to 0.4 ml, up to 0.5 ml, up to 0.7 ml, up to 1.0 ml or up to 2.0 ml.

Due to the labelling of the amplified DNA, wherever sample molecules interact with probe molecules on the surface of the microarray, a reporter reagent binds the label and produces visible signals which may be detected by a detection device. The interacting probe and sample molecules are identified by the location of the signal on the surface of the microarray. In the particular case where sample DNA molecules are labelled with biotin, the reporter agent can be horseradish peroxidase covalently joined to streptavidin. The latter binds specifically to biotin, and the peroxidase triggers the precipitation of substrates like tetramethylbenzidine (TMB). Any other reaction that results in a precipitate on array elements, and that can be used to detect the interaction between target and probe molecules according to the present invention may be used.

The present invention further relates to claim 6

The first mixture of amplification primers may additionally comprise primers L6 (SEQ ID Nº 7) and L7 (SEQ ID Nº 8).

Additionally, the kit of the present invention may further allow detection of Influenza A, Influenza B, Influenza C, PIV 1, PIV 2, PIV 3, PIV 4A, 4B, Adenovirus, hRSV A, hRSV B, Coronavirus229, Echovirus 30, Rhinovirus and hBoV, in a test sample, the kit preferably comprising:
- Pairs of primers of SEQ ID Nº 14-24 for PIV 1, PIV 2, PIV 3, PIV 4A, 4B, hRSV A and Coronavirus type 229, within the first amplification mixture; and
- pairs of primers of SEQ ID Nº 25-38 for Influenza A, Influenza B, Influenza C, hRSV B, Adenovirus, Echovirus 30, Rhinovirus and hBoV, within the second amplification mixture.

Further to the amplification mixtures, the kit of the invention may further comprise:
i) an array vessel or set of array vessels, each comprising a microarray wherein target-specific probes are provided; and
ii) reagents for use in visualising hybridisation of nucleic acids to the probes of the microarray.

For example, one or more amplification tubes further comprise one or more internal controls. In a preferred embodiment, the protocol of the present invention includes an internal control of the nucleic acids extraction step. In another preferred embodiment, the protocol of the present invention includes an internal control of the nucleic acid amplification step.

A preferred internal control would be a DNA plasmid that would be amplifiable with a pair of amplification primers. In a most preferred embodiment, one or more Multiplex tubes comprise an Internal Control DNA in the form of a DNA plasmid, and primers RTS (GCTTGGGCGTGTCTCAAAATCT, SEQ ID NO 39) and
RTA (GTCGCCACGGTTGATGAGAGCT, SEQ ID NO 40) for its amplification. Particularly preferred conditions are:

| Multiplex Reactants | volume (µl) |
|---|---|
| Internal Control DNA, 10⁴ copies/µl | 1 |
| RTS 20 µM | 0.5 |
| RTA 20µM | 0.5 |

In a final reaction volume of 50 µl.

Further particularly preferred conditions are:

| Multiplex Reactans | Volume (µl) |
|---|---|
| Internal Control DNA, 10⁴ copies/µl | 4 |
| RTS 20 µM | 0.5 |
| RTA 20µM | 0.5 |

In a final reaction volume of 50 µl.

Either one or both amplification primers RTS/ RTA may be labelled.

The amplification product corresponding to the internal control DNA is a DNA fragment of 885 bp.

In a particular embodiment, the microarray comprises probes for specific detection of the internal control amplifiable with amplification primers RTS and RTA. In particular,
CI 1 5' (CAGCTGGCACGACAGGTTTCCCGACTGG, SEQ 10 NO 41),
CI 1 3' (TTGAAGTGGTGGCCTAACTACGG, SEQ ID NO 42) and
CI 2 5' (CGTTCCACTGAGCGTCAGACCC, SEQ 10 NO 43) may be used.

A further preferred internal control would be an RNA fragment that would preferably be added to the test sample prior to the nucleic acid extraction. Another preferred internal control would be an RNA fragment that would preferably be added to the nucleic acids after extraction from the test sample. In a more preferred embodiment, the RNA fragment would be present within the vessel containing the amplification reactants, prior to incubation with the nucleic acids obtained from the test sample.

In a more preferred embodiment, the RNA fragment could be obtained by RNA transcription of a plasmid. In another preferred embodiment the RNA fragment could be obtained by chemical synthesis.

Particularly preferred target-specific probes corresponding to hMPV amplification products described herein comprise sequences selected from the group comprising SEQ ID Nº 9 to 13, their complements and equivalents thereof. In a most preferred embodiment, the target-specific probes corresponding to hMPV amplification products consist of SEQ ID Nº 9 to 13.

The probes can be obtained by different methods, such as chemical synthesis (e. g. by the conventional phosphotriester method) or genetic engineering techniques, for example by molecular cloning of recombinant plasmids in which corresponding nucleotide sequences have been inserted and can be later obtained by digestion with nucleases.

Specific probes may be designed using the nucleic acid alignment program Oligo 6. The parameters of Tm and G/C ratio are analysed in all cases, and secondary-structure formation was avoided. Preferred probes are those with the same Tm under the salt concentration which is used in the hybridisation step. In a preferred embodiment, probes are selected to bind to their corresponding target sequences under the same hybridisation conditions. The skilled person will be aware of other ways in which specific probes may be designed.

The probes can be selected so that they do not hybridize with genomic DNA, and do not hybridize in an unspecific way with amplified fragments corresponding to other viruses.

One or more probes described herein can be provided on a solid support.

In another preferred embodiment, two or more target-specific probes for the same target sequence are provided on the solid support, in order to improve detection of the viral agents of interest.

Said probes or mixtures of probes may be immobilized in a single location of the solid support, preferably in two distinct locations of the solid support and more preferably in three distinct locations of the solid support.

In a preferred embodiment, the probes are provided on a solid support located within an array vessel.

In another embodiment, the probes are provided on a solid support located within an array strip.

In a more preferred embodiment, the array strip has 8 array vessels.

In one preferred embodiment, the solid support is a coated glass slide.

In another preferred embodiment, the solid support is the bottom of an array vessel, the array vessel being either an individual array tube, or a component of a strip of vessels or set of strip of vessels, such as a microtiter plate.

In a preferred embodiment, the interactions taking place between DNA amplified with the pairs of primers of the present invention, and corresponding detection probes, take place on an individual array vessel, on a strip of vessels, or on a set of strips of vessels.

In a preferred embodiment, visualization of such interactions consists of the following steps:
■ First, the image of the array is captured using an optical device,
■ Then the image is analysed,
■ Finally, a report containing an interpretation of the result is provided.

Preferably, the image is analysed by means of appropriate software.

Any device suitable for this processing can be used.

We also describe the use of the above-mentioned method and kit for the detection and identification, if present in a test sample, of one or more viral agents selected from the group comprising hMPV, Influenza A, Influenza B, Influenza C, PIV 1, PIV 2, PIV 3, PIV 4A, 4B, Adenovirus, hRSV A, hRSV B, Coronavirus229, Echovirus 30, Rhinovirus and hBoV.

It has been confirmed, as in the Examples provided in the present invention, that the nucleic acids of SEQ ID Nº 1 to 6, when used as amplification primers in hMPV amplification, provide better values of sensitivity of detection of hMPV than the pair of primers L6/L7 of the state of the art. This is so, even in the presence of amplification primers corresponding to other respiratory viruses.

Further aspects of the present invention correspond to claim 12 and claim 13. we also describe (1) a nucleic acid amplification mixture comprising, as amplification primers, nucleic acids comprising SEQ ID Nº 1 and 2, and/or nucleic acids comprising SEQ ID Nº 3 and 4, and/or nucleic acids comprising SEQ ID Nº 5 and 6, or their complements and equivalents thereof; (ii) Amplification fragments obtainable with the pairs of primers of SEQ ID Nº 1 and 2, 3 and 4; and 5 and 6; and (iii) A method for producing nucleic acid sequences comprising SEQ ID Nº 1-6 their complements and equivalents thereof, wherein the nucleic acid sequences may be labelled. Preferably this label is biotin.

We also describe probes for the detection of hMPV, comprising a sequence selected from the group comprising SEQ ID Nº 9 to 13, their complements and equivalents thereof.

### Examples

The examples provided below merely illustrate the invention and in no way limit the scope of the accompanying claims.

### EXAMPLE 1.

200 µl of a nasopharyngeal wash test sample, were subjected to the automatic extraction system NucliSENS easyMAG of BioMerieux, followed by resuspension in 20 µl RNase-free water.

5 µl of the total 20 µl were added to two different vessels, each containing amplification reactants, in a final volume of 50 µl. Amplification reactants included amplification primers of the state of the art L6 and L7 (SEQ ID Nº 7 and 8) in the absence ("Tube -") or presence ("Tube +") of the pair of amplification primers L6.2 and L7.2 (SEQ ID Nº 1 and 2, respectively, of Table I).

The amplification mixture corresponding to "Tube +" was as follows:

| MIX 1X | Volume (µl) |
|---|---|
| RNAse free water | 7,6 |
| 5X QUIAGEN buffer | 10 |
| dNTP mix 10mM | 2 |
| 5X Q-solution | 10 |
| QUIAGEN enzime mix | 2 |
| B-L6 (40µM) | 1 |
| B-L7 (40µM) | 1 |
| B-L6.2 (40µM) | 1 |
| B-L7.2 (40µM) | 1 |
| B-3PIV24 (40µM) | 1 |
| B-4PIV4 (40µM) | 0,8 |
| B-4PIV2 (40µM) | 0,8 |
| B-RSVA S15 (40µM) | 0,7 |
| B-RSVA (2) 3' (40µM) | 0,7 |
| B-1PIV13+ (40) | 1 |
| B-PIV1 A218 (40µM) | 1 |
| B-1HCoV (40µM) | 0,5 |
| B-Cor229-214 (40µM) | 0,5 |
| B-PIV3 S118 (40µM) | 0,7 |
| B-PIV3 A313 (40µM) | 0,7 |
| B-RTS 6/9/07 (20µM) | 0,5 |
| B-RTA 6/9/07 (20µM) | 0,5 |
| Internal control (10e4 copies/µl) | 1 |
| Sample | 5 |

| | |
|---|---|
| Prefix "B-" means biotin-labeled. | |

The amplification mixture corresponding to "Tube -" was exactly as that of "Tube +", with the exception that amplification primers L6.2 and L7.2 were substituted by RNase free water.

Amplification conditions were:

| | |
|---|---|
| 1 cycle | 45ºC 45min |
| | 95ºC 15min |
| 45 cycles | 95ºC 30 sec |
| | 50ºC 1:30 min |
| | 68ºC 1 min |
| 1 cycle | 68ºC 10 min |
| Final 4ºC (optional) | |

Visualization of the amplification products corresponding to four different test samples amplified both with "Tube -" and "Tube +", by agarose gel electrophoresis, is displayed in Figure 1.

Experimental conditions previously described have proven equally efficient for the testing of samples constituted by pharyngeal exudates, nasopharyngeal exudates, or bronco-alveolar washes.

### EXAMPLE 2.

5 µl of a total of 20 µl, obtained from the test samples S7 (hMPV-A positive) and S2 (hMPV-B positive), following the same procedure as in EXAMPLE 1, were added to reactants of Multiplex 1 or Multiplex 2, in a final volume of 50 µl.

Conditions of Multiplex 1 and 2 were as follows:

| Reactants Multiplex 1 | Initial concentration | Volume (µl) |
|---|---|---|
| RNase-free water | | Up to 50 µl |
| 5X QIAGEN ONE STEP RT-PCR BUFFER | 5X | 10 |
| 5X Q SOLUTION | | 10 |
| B-L6 | 40 µM | 1 |
| B-L7 | 40 µM | 1 |
| B-L6.2 | 40 µM | 1 |
| B-L7.2 | 40 µM | 1 |
| B-1PIV13 | 40 µM | 1 |
| B-Piv1-A-218 | 40 µM | 1 |
| B-3PIV24 | 40 µM | 0.8 |
| B-4PIV2 | 40 µM | 0.8 |
| B-Piv3-S-118 | 40 µM | 0.7 |
| B-Piv-3-A-313 | 40 µM | 0.7 |
| B-4PIV4 | 40 µM | 0.8 |
| B-1HCoV | 40 µM | 0.5 |
| B-Cor229-A-214 | 40 µM | 0.5 |
| B-RSVA-S-15 | 40 µM | 0.7 |
| B-RSVA (2) 3' | 40 µM | 0.7 |
| RTS-B | 20 µM | 0.5 |
| RTA-B | 20 µM | 0.5 |
| dNTPs MIX 10 mM | 10 mM | 2 |
| Internal Control | 10e4 copies/µl | 1 |
| Enzyme | | 2 |
| Sample | | 5 |
| TOTAL | | 50 |

| | | |
|---|---|---|
| Prefix "B-" means biotin-labeled. | | |

| Reactants Multiplex 2 | Initial concentration | Volume (µl) |
|---|---|---|
| RNase-free water | | Up to 50 µl |
| 5X QIAGEN ONE STEP RT-PCR BUFFER | 5X | 10 |
| 5X Q SOLUTION | | 10 |
| B-SA | 40 µM | 0.1 |
| B-SB | 40 µM | 0.1 |
| B-AA | 40 µM | 0.1 |
| B-AB | 40 µM | 0.1 |
| B-NPAC11+ | 40 µM | 1 |
| B-FluA-A-241 | 40 µM | 0.8 |
| B-NPB1+ | 40 µM | 0.5 |
| B-FluB-A-136 | 40 µM | 0.5 |
| B-FluC-S-122 | 40 µM | 0.3 |
| B-FluC-A-217 | 40 µM | 0.3 |
| B-ADV1F+ | 40 µM | 0.8 |
| B-ADV2R- | 40 µM | 0.8 |
| B-HER1 | 40 µM | 0.2 |
| HER4 | 40 µM | 0.2 |
| B-RSVB-541 | 40 µM | 0.5 |
| B-RSVB 3' | 40 µM | 0.5 |
| B-NS1 S-1545 | 40 µM | 0.25 |
| B-NS1 A-1835 | 40 µM | 0.25 |
| B-RTS | 20 µM | 0.5 |
| B-RTA | 20 µM | 0.5 |
| dNTPs MIX 10 mM | 10 mM | 2 |
| Internal Control | 10e4 copies/µl | 1 |
| Enzyme | | 2 |
| Sample | | 5 |
| TOTAL | | 50 |

| | | |
|---|---|---|
| Prefix "B-" means biotin-labeled. | | |

Amplification conditions were the same for both Multiplex 1 and 2:

| | |
|---|---|
| 1 cycle | 45ºC 45min |
| | 95ºC 15min |
| 45 cycles | 95ºC 30 sec |
| | 50ºC 1:30 min |
| | 68ºC 1 min |
| 1 cycle | 68ºC 10 min |
| Final 4°C (optional) Final 4ºC (optional) | |

Single-stranded oligo- or polynucleotides were obtained from the corresponding amplification products, and allowed to hybridise with a plurality of target-specific probes provided on a solid support, comprised within an Array Tube.

Amplification products obtained with the pair of amplification primers L6 and L7 in the absence and presence of L6.2 and L7.2, were denatured and hybridized with newly designed probes HMNVA-S37 and HMNVB-S38 (SEQ ID Nº 9 and 10, respectively), which respectively detect genotypes A and B of hMPV.

Hybridization of above-mentioned amplification products with a generic probe for hMPV provided the same result.

Amplification products obtained with the pairs of amplification primers SA/AA and SB/AB, were denatured and hybridized with newly designed probes F249B, F256B and F256A (SEQ ID Nº 11, 12 and 13, respectively), which respectively detect genotypes B, B and A of hMPV.

Representative examples of the images corresponding to a hMPV-A positive sample (Sample S7 of Table II) and to a hMPV-B positive sample (Sample S2 of Table II), are displayed in Figure 2. Amplification conditions were those of Multiplex 1 and 2, and hybridization probes were: HMNVA-S37 and HMNVB-S38, in the case of amplification products obtained in Multiplex 1; And F249B, F256B and F256A, in the case of amplification products obtained in Multiplex 2.

## Claims

1. A method for the detection of hMPV in a test sample comprising:
a) dividing the test sample into two or more samples;
b) contacting a first sample with a first mixture of amplification primers, said mixture comprising first and second nucleic acid sequences comprising SEQ ID N° 3 and 4 respectively and third and fourth nucleic acid sequences comprising SEQ ID NO 5 and 6 respectively, and/ or their complements or sequences that differ from sequences of SEQ ID N° 3, 4, 5 and 6 by 1 mutation thereof;
c) contacting a second sample with a second mixture of amplification primers, said mixture comprising first and second nucleic acid sequences comprising SEQ ID N° 1 and 2 respectively, and/ or their complements or sequences that differ from sequences of SEQ ID N° 1 and 2 by 1 mutation thereof;
d) subjecting the first and second samples, mixed with the amplification primers of steps b) and c), respectively, to amplification reactions being intended to amplify target sequences present in the sample;
e) obtaining single-stranded oligo- or polynucleotides from any amplification products, and allowing such single-stranded oligo- or polynucleotides to hybridise with a plurality of target-specific probes, and
f) detecting hybridised oligo- or polynucleotides.

2. The method of claim 1 wherein single-stranded oligo- or polynucleotides are obtained by denaturing any double-stranded oligo- or polynucleotides present in the amplification products.

3. The method of claims 1 and 2 wherein the plurality of target-specific probes comprises one or more sequences selected from the group comprising SEQ ID N° 9, 10, 11, 12 and 13, their complements and sequences that differ from them by 1 mutation thereof.

4. The method of claims 1 to 3, wherein:
- amplification products obtained with the first mixture of amplification primers comprising first and second nucleic acid sequences comprising SEQ ID N° 3 and 4 respectively and third and fourth nucleic acid sequences comprising SEQ ID N° 5 and 6 respectively, or their complements and sequences that differ from them by 1 mutation thereof, are hybridised with target-specific probes comprising one or more probes selected from the group comprising SEQ ID N° 11 to 13, and
- amplification products obtained with the second mixture of amplification primers comprising first and second nucleic acid sequences comprising SEQ ID NO 1 and 2 respectively, or their complements and sequences that differ from them by 1 mutation thereof, are hybridised with target-specific probes comprising one or more probes selected from the group comprising SEQ ID N° 9 and 10.

5. The method of claims 1 to 4 wherein the second mixture of amplification primers further comprises primers of SEQ ID N° 7 and 8.

6. A kit for the detection and identification in a test sample of hMPV, wherein said kit comprises two or more amplification mixtures,
- A first mixture comprising first and second nucleic acid sequences comprising sequences SEQ ID N° 3 and 4 respectively, and third and fourth nucleic acid sequences comprising sequences SEQ ID N° 5 and 6 respectively, or their complements and sequences that differ from sequences of SEQ ID N° 3, 4, 5 and 6 by 1 mutation thereof, as components of a pair of amplification primers for hMPV; and
- A second mixture comprising first and second nucleic acid sequences comprising sequences SEQ ID N° 1 and 2 respectively, or their complements and sequences that differ from sequences of SEQ ID N° 1 and 2 by 1 mutation thereof, as components of other pairs of amplification primers for hMPV.

7. The kit of claim 6, wherein the second mixture of amplification primers further comprises primers comprising the nucleic acid sequences of SEQ ID N° 7 and 8.

8. The kit according to claims 6 and 7, further allowing detection of Influenza A, Influenza B, Influenza C, PIV 1, PIV 2, PIV 3, PIV 4A, 4B, Adenovirus, hRSV A, hRSV B, Coronavirus229, Echovirus 30, Rhinovirus and hBoV, in a test sample, the kit comprising:
- Pairs of primers comprising nucleic acid sequences selected from the group comprising SEQ ID N° 25-38 for Influenza A, Influenza B, Influenza C, hRSV B, Adenovirus, Echovirus 30, Rhinovirus and hBoV, within the first amplification mixture; and
- Pairs of primers comprising nucleic acid sequences selected from the group comprising SEQ ID NO 14-24 for PIV 1, PIV 2, PIV 3, PIV 4A, 4B, hRSV A and Coronavirus type 229, within the second amplification mixture.

9. The kit according to claims 6 to 8, further comprising:
i) an array vessel or set of array vessels, each comprising a microarray wherein target-specific probes are provided; and
ii) reagents for use in visualising hybridisation of nucleic acids to the probes of the microarray.

10. The kit of claim 9, wherein the target-specific probes comprise one or more sequences selected from the group comprising SEQ ID NO 9 to 13, their complements and sequences that differ from them by 1 mutation thereof, as target-specific probes for hMPV.

11. The kit of claims 9 and 10, wherein target-specific probes are comprised on an individual array vessel, or on a set of array vessels.

12. A set of primers comprising nucleic acid sequences SEQ ID N° 1 to 6.

13. A set of probes consisting of at least one probe selected from SEQ ID N° 9 and 10, and at least one probe selected from SEQ ID N° 11 to 13.

## Patentansprüche

1. Verfahren zum Nachweis von hMPV (humanem Metapneumovirus) in einer Testprobe, wobei das Verfahren aufweist:
a) Unterteilen der Testprobe in zwei oder mehrere Proben;
b) Inkontaktbringen einer ersten Probe mit einem ersten Gemisch von Amplifikationsprimem, wobei das Gemisch aufweist: erste bzw. zweite Nucleinsäuresequenzen, die SEQ ID Nr.: 3 bzw. 4 enthalten, und dritte bzw. vierte und Nucleinsäuresequenzen, die SEQ ID Nr.: 5 bzw. SEQ ID Nr.: 6 enthalten, und/oder deren Komplemente oder Sequenzen, die sich durch 1 Mutation von den Sequenzen SEQ ID Nr.: 3, 4, 5 und 6 unterscheiden;
c) Inkontaktbringen einer zweiten Probe mit einem zweiten Gemisch von Amplifikationsprimern, wobei das Gemisch aufweist: erste bzw. zweite Nucleinsäuresequenzen, die SEQ ID Nr.: 1 bzw. 2 enthalten, und/oder deren Komplemente oder Sequenzen, die sich durch 1 Mutation von den Sequenzen SEQ ID Nr.: 1 und 2 unterscheiden;
d) Durchführen von Amplifikationsreaktionen, die in der Probe anwesende Targetsequenzen amplifizieren sollen, an den ersten bzw. zweiten Proben, die mit den Amplifikationsprimern der Schritte b) bzw. c) vermischt sind;
e) Gewinnung von einzelsträngigen Oligo- oder Polynucleotiden aus beliebigen Amplifikationsprodukten und Hybridisieren derartiger einzelsträngiger Oligo- oder Polynucleotide mit einer Vielzahl von targetspezifischen Sonden, und
f) Nachweis von hybridisierten Oligo- oder Polynucleotiden.

2. Verfahren nach Anspruch 1, wobei einzelsträngige Oligo- oder Polynucleotide durch Denaturieren beliebiger, in den in den Amplifikationsprodukten anwesender doppelsträngiger Oligo- oder Polynucleotide gewonnen werden.

3. Verfahren nach Anspruch 1 und 2, wobei die Vielzahl von targetspezifischen Sonden eine oder mehrere Sequenzen aufweist, die aus der Gruppe ausgewählt sind, die aus SEQ ID Nr.: 9, 10, 11, 12 und 13, deren Komplementen und Sequenzen besteht, die sich durch 1 Mutation davon unterscheiden.

4. Verfahren nach einem der Ansprüche 1 bis 3, wobei
- Amplifikationsprodukte, die mit dem ersten Gemisch von Amplifikationsprimem gewonnen werden, das erste bzw. zweite Nucleinsäuresequenzen, die SEQ ID Nr.: 3 bzw.4 enthalten, und dritte bzw. vierte Nucleinsäuresequenzen, die SEQ ID Nr.: 5 bzw. SEQ ID Nr.: 6 enthalten, oder deren Komplemente oder Sequenzen aufweist, die sich durch 1 Mutation davon unterscheiden, mit targetspezifischen Sonden hybridisiert werden, die eine oder mehrere Sonden aufweisen, die aus der Gruppe ausgewählt sind, die aus den SEQ ID Nrn. 11 bis 13 besteht, und
- Amplifikationsprodukte, die mit dem zweiten Gemisch von Amplifikationsprimern gewonnen werden, das erste bzw. zweite Nucleinsäuresequenzen, die SEQ ID Nr.: 1 bzw. 2 enthalten, oder deren Komplemente oder Sequenzen aufweist, die sich durch 1 Mutation davon unterscheiden, mit targetspezifischen Sonden hybridisiert werden, die eine oder mehrere Sonden aufweisen, die aus der Gruppe ausgewählt sind, die aus den SEQ ID Nrn. 9 und 10 besteht.

5. Verfahren nach einem der Ansprüche 1 bis 4, wobei das zweite Gemisch von Amplifikationsprimern ferner die Primer gemäß SEQ ID Nr.: 7 und 8 aufweist.

6. Kit für den Nachweis und zur Identifikation von hMPV in einer Testprobe, wobei der Kit zwei oder mehrere Amplifikationsgemische aufweist,
- ein erstes Gemisch, das erste bzw. zweite Nucleinsäuresequenzen, die Sequenzen gemäß SEQ ID Nr.: 3 bzw. 4 enthalten, und dritte bzw. vierte Nucleinsäuresequenzen, die Sequenzen gemäß SEQ ID Nr.: 5 bzw. 6 enthalten, oder deren Komplemente und Sequenzen, die sich durch 1 Mutation von den Sequenzen SEQ ID Nr.: 3, 4, 5 und 6 unterscheiden, als Komponenten eines Amplifikationsprimerpaars für hMPV aufweist; und
ein zweites Gemisch, das erste bzw. zweite Nucleinsäuresequenzen, die Sequenzen gemäß SEQ ID Nr.: 1 bzw. 2 enthalten, oder deren Komplemente und Sequenzen, die sich durch 1 Mutation von den Sequenzen SEQ ID Nr.: 1 und 2 unterscheiden, als Komponenten anderer Amplifikationsprimerpaare für hMPV aufweist.

7. Kit nach Anspruch 6, wobei das zweite Gemisch von Amplifikationsprimern ferner Primer enthält, welche die Nucleinsäuresequenzen gemäß SEQ ID Nr.: 7 und 8 aufweisen.

8. Kit nach einem der Ansprüche 6 und 7, der ferner den Nachweis von Influenza A, Influenza B, Influenza C, PIV 1, PIV 2, PIV 3, PIV 4A, 4B, Adenovirus, hRSV A, hRSV B, Coronavirus 229, Echovirus 30, Rhinovirus und hBoV in einer Testprobe ermöglicht, wobei der Kit aufweist:
- Primerpaare, die Nucleinsäuresequenzen aufweisen, die aus der Gruppe ausgewählt sind, die aus SEQ ID Nr.: 25-38 für Influenza A, Influenza B, Influenza C, hRSV B, Adenovirus, Echovirus 30, Rhinovirus und hBoV besteht; innerhalb des ersten Amplifikationgemischs; und
- Primerpaare, die Nucleinsäuresequenzen aufweisen, die aus der Gruppe ausgewählt sind, die aus SEQ ID Nr.: 14-24 für PIV 1, PIV 2, PIV 3, PIV 4A, 4B, hRSV A und Coronavirus Typ 229 besteht, innerhalb des zweiten Amplifikationgemischs.

9. Kit nach einem der Ansprüche 6 bis 8, der ferner aufweist:
i) einen Array-Behälter oder eine Gruppe von Array-Behältern, die jeweils ein Mikroarray aufweisen, in dem targetspezifische Sonden bereitgestellt werden; und
ii) Reagenzien zur Verwendung beim Sichtbarmachen der Hybridisierung von Nucleinsäuren an die Sonden des Mikroarrays.

10. Kit nach Anspruch 9, wobei die targetspezifischen Sonden eine oder mehrere Sequenzen, die aus der Gruppe ausgewählt sind, die aus SEQ ID Nr.: 9 bis 13, deren Komplementen und Sequenzen besteht, die sich durch 1 Mutation davon unterscheiden, als targetspezifische Sonden für hMPV aufweisen.

11. Kit nach einem der Ansprüche 9 und 10, wobei targetspezifische Sonden auf einem einzelnen Array-Behälter oder auf einer Gruppe von Array-Behältern gebildet werden.

12. Gruppe von Primern, welche die Nucleinsäuresequenzen SEQ ID Nr.: 1 bis 6 aufweisen.

13. Gruppe von Sonden, die aus mindestens einer unter SEQ ID Nr.: 9 und 10 ausgewählten Sonde und mindestens einer unter SEQ ID Nr.: 11 bis 13 ausgewählten Sonde besteht.

## Revendications

1. Procédé pour la détection de MPVh dans un échantillon de test consistant:
a) à diviser l'échantillon de test en deux échantillons en plus;
b) à mettre en contact un premier échantillon avec un premier mélange d'amorces d'amplification, ledit mélange comprenant une première et une deuxième séquences d'acide nucléique comprenant les SEQ ID N° 3 et 4 respectivement et une troisième et une quatrième séquences d'acide nucléique comprenant les SEQ ID N° 5 et 6 respectivement et/ou leurs compléments ou des séquences qui diffèrent des séquences des SEQ ID N° 3, 4, 5 et 6 de 1 mutation de celles-ci;
c) à mettre en contact un deuxième échantillon avec un deuxième mélange d'amorces d'amplification, ledit mélange comprenant une première et une deuxième séquences d'acide nucléique comprenant les SEQ ID N° 1 et 2 respectivement et/ou leurs compléments ou des séquences qui diffèrent des séquences des SEQ ID N° 1 et 2 de 1 mutation de celles-ci;
d) à soumettre le premier et le deuxième échantillon, mélangés avec les amorces d'amplification des étapes b) et c), respectivement, à des réactions d'amplification qui sont destinées à amplifier des séquences cibles présentes dans l'échantillon;
e) à obtenir des oligo- ou des polynucléotides à brin simple à partir de tous produits d'amplification et à permettre à ces oligo- ou polynucléotides à brin simple de s'hybrider avec une pluralité de sondes spécifiques à une cible, et
f) à détecter des oligo- ou polynucléotides hybridés.

2. Procédé selon la revendication 1, dans lequel les oligo- ou polynucléotides à simple brin sont obtenus en dénaturant tous oligo- ou polynucléotides à brin double présents dans les produits d'amplification.

3. Procédé selon les revendications 1 et 2, dans lequel la pluralité de sondes spécifiques à une cible comprend une ou plusieurs séquences choisies dans le groupe comprenant les SEQ ID N° 9, 10, 11, 12 et 13, leurs compléments et des séquences qui diffèrent de celles-ci de 1 mutation de celles-ci.

4. Procédé selon les revendications 1 à 3, dans lequel:
- les produits d'amplification, obtenus avec le premier mélange d'amorces d'amplification comprenant une première et une deuxième séquences d'acide nucléique comprenant les SEQ ID N° 3 et 4 respectivement et une troisième et une quatrième séquences d'acide nucléique comprenant les SEQ ID N° 5 et 6 respectivement ou leurs compléments et des séquences qui diffèrent de celles-ci de 1 mutation de celles-ci, sont hybridés avec des sondes spécifiques à une cible comprenant une ou plusieurs sondes choisies dans le groupe comprenant les SEQ ID N° 11 à 13, et
- les produits d'amplification, obtenus avec le deuxième mélange d'amorces d'amplification comprenant une première et une deuxième séquences d'acide nucléique comprenant les SEQ ID N° 1 et 2 respectivement ou leurs compléments et des séquences qui diffèrent de celles-ci de 1 mutation de celles-ci, sont hybridés avec des sondes spécifiques à une cible comprenant une ou plusieurs sondes choisies dans le groupe comprenant les SEQ ID N° 9 et 10.

5. Procédé selon les revendications 1 à 4, dans lequel le deuxième mélange d'amorces d'amplification comprend en outre des amorces des SEQ ID N° 7 et 8.

6. Kit pour la détection et l'identification dans un échantillon de test de MPVh, où ledit kit comprend deux mélanges d'amplification ou plus,
- un premier mélange comprenant une première et une deuxième séquences d'acide nucléique comprenant les séquences SEQ ID N° 3 et 4 respectivement et une troisième et une quatrième séquences d'acide nucléique comprenant les séquences SEQ ID N° 5 et 6 respectivement ou leurs compléments et des séquences qui diffèrent des séquences des SEQ ID N° 3, 4, 5 et 6 de 1 mutation de celles-ci, en tant que composants d'une paire d'amorces d'amplification pour MPVh; et
- un deuxième mélange comprenant une première et une deuxième séquences d'acide nucléique comprenant les séquences SEQ ID N° 1 et 2 respectivement ou leurs compléments et des séquences qui diffèrent des séquences des SEQ ID N° 1 et 2 de 1 mutation de celles-ci, en tant que composants d'autres paires d'amorces d'amplification pour MPVh.

7. Kit selon la revendication 6, dans lequel le deuxième mélange d'amorces d'amplification comprend en outre des amorces comprenant les séquences d'acide nucléique des SEQ ID N° 7 et 8.

8. Kit selon les revendications 6 et 7, permettant en outre la détection de la grippe A, de la grippe B, de la grippe C, de PIV 1, de PIV 2, de PIV 3, de PIV 4A, 4B, d'adénovirus, de RSVh A, de RSVh B, du coronavirus 229, de l'Echovirus 30, de rhinovirus et de BoVh, dans un échantillon de test, le kit comprenant:
- des paires d'amorces comprenant des séquences d'acide nucléique choisies dans le groupe comprenant les SEQ ID N° 25-38 pour la grippe A, la grippe B, la grippe C, RSVh B, l'adénovirus, l'Echovirus 30, le rhinovirus et BoVh, dans le premier mélange d'amplification; et
- des paires d'amorces comprenant des séquences d'acide nucléique choisies dans le groupe comprenant les SEQ ID N° 14-24 pour PIV 1, PIV 2, PIV 3, PIV 4A, 4B, RSVh A et le coronavirus de type 229, dans le deuxième mélange d'amplification.

9. Kit selon les revendications 6 à 8, comprenant en outre:
i) un récipient à réseau ou un jeu de récipients à réseau, chacun comprenant un micro-réseau dans lequel des sondes spécifiques à une cible sont fournies; et
ii) des réactifs pour une utilisation dans la visualisation de l'hybridation d'acides nucléiques avec les sondes du micro-réseau.

10. Kit selon la revendication 9, dans lequel les sondes spécifiques à une cible comprennent une ou plusieurs séquences choisies dans le groupe comprenant les SEQ ID N° 9 à 13, leurs compléments et des séquences qui diffèrent de celles-ci de 1 mutation de celles-ci, en tant que sondes spécifiques à une cible pour MPVh.

11. Kit selon les revendications 9 et 10, dans lequel des sondes spécifiques à une cible sont comprises sur un récipient à réseau individuel ou sur un jeu de récipients à réseau.

12. Jeu d'amorces comprenant les séquences d'acide nucléique SEQ ID N° 1 à 6.

13. Jeu de sondes constitué d'au moins une sonde choisie parmi les SEQ ID N° 9 et 10 et d'au moins une sonde choisie parmi les SEQ ID N° 11 à 13.
